# EUROPEAN PATENT APPLICATION

(11) **EP 4 356 936 A1**
(43) Date of publication of application: **24.04.2024**
(21) Application number: 22825086.6
(22) Date of filing: 17.06.2022
(51) Int. Cl.: A61L 27/38, A61F 2/18

(54) **SKELETAL STRUCTURE FOR ARTIFICIAL PINNA AND ARTIFICIAL CARTILAGINOUS TISSUE, AND ARTIFICIAL PINNA USING SAME**

(30) Priority: 17.06.2021 JP 2021100818
(71) Applicant: Kinki University, Higashiosaka-shi, Osaka 577-8502 (JP)
(72) Inventor: ISOGAI Noritaka, Osaka-Sayama City, Osaka 589-8511 (JP); TERAMURA Takeshi, Osaka-Sayama City, Osaka 589-8511 (JP); MASUTANI Kazunari, Kyoto-shi, Kyoto 612-8374 (JP); MITACHI Hiroshi, Kyoto-shi, Kyoto 604-8823 (JP); YOTSUYANAGI Takatoshi, Sapporo-shi, Hokkaido 060-8556 (JP)
(74) Representative: Grünecker Patent- und Rechtsanwälte PartG mbB
(86) International application number: PCT/JP2022/024326
(87) International publication number: WO 2022/265098

(57) **Abstract**

In the reconstruction of a lost pinna, an implant-type regeneration therapy is performed. In this case, skeletal structures using bioabsorbable materials have been proposed as a scaffolding material for the cells, and these skeletal structures are expected to be flexible as well as strong while being easily deformed and possessing a shape-restoring ability.

A skeletal structure for an artificial pinna includes a base portion formed in a flat shape with a fibrous structural body using a bioabsorbable material, and a helix portion and an antihelix portion disposed on the front surface of the base portion. In the skeletal structure, the base portion is provided with a two-dimensional elastic structure, and the helix portion and the antihelix portion are provided with a three-dimensional fiber structure formed with the fibrous structural bodies. Thus, a flexible skeletal structure can be provided.

## Description

### Technical Field

The present invention relates to a skeletal structure for an artificial pinna used to reconstruct a lost pinna.

### Background Art

It was said that cartilaginous tissue of the nose or external ear has a complex three-dimensional structure and reconstruction of this tissue is one of the major challenges in plastic surgery. In particular, the ear is the cartilaginous tissue with the most complex structure. Pinna reconstruction is required in cases of tissue sacrifice associated with congenital deformities, microtia, or melanoma, and injuries caused by accidents, severe burns, or the like.

The Tanzer method is commonly used for the pinna reconstruction. In this method, a frame (external shape) of the pinna is created using costal cartilages taken from the patient's 6th, 7th, and 8th ribs, and is implanted subcutaneously in the temporal region of the head. After several months, the implanted pinna frame and skin are made erect, and skin grafts are performed on the posterior surface of the pinna and the temporal region of the head.

However, this method is highly invasive and imposes a heavy burden on the patient. Thus, an implant-type regenerative therapeutic method has been developed in which the cells are cultured in vitro for a certain period of time and then implanted into the body. However, in this method, if the cultured cells, when implanted into the body, fail to maintain a stable tissue shape, the subsequent tissue regeneration is unlikely to be successful.

Thus, an artificial pinna made from a plastic (polyethylene) has been implanted as a scaffolding material. However, this plastic exhibits low biocompatibility and thus occasionally causes clinical issues such as inflammation and protrusion due to foreign body reactions.

To address this problem, there has been proposed an artificial pinna that is made using a bioabsorbable material as a scaffolding material for the cells to be implanted. PTL 1 discloses that an artificial pinna is formed using a hydroxyapatite/chondroitin sulfate-collagen complex or a hydroxyapatite-collagen complex as a cell scaffolding material. This material is a porous carrier and is expected to be elastic and mechanically strong.

As a method of adjusting elasticity or mechanical strength, a method of using a composite material can be considered. PTL 2 discloses that an artificial pinna that resembles the actual pinna is obtained by assigning the roles required for the artificial pinna to multiple elements such as a skin simulation layer (201), a hydrogel layer (202), a hydrogel microcapsule layer (203), and a skeletal simulation layer (204), and putting these elements together.

### Citation List

### Patent Literature

PTL 1: Japanese Patent Application Laid-Open No. 2012-000262
PTL2: Chinese Patent Application Publication No. 104490491
PTL3: International Publication No. 2013/081103

### Summary of Invention

### Technical Problem

In the artificial pinna of PTL 2, a part corresponding to the skeleton is formed by a planar mesh structural body. Thus, although it is relatively flexible in the twisting direction, it exhibits stress against a force from the in-plane direction, making it difficult to obtain flexibility in this direction. There is another problem in that the artificial pinna does not have an antihelix portion and thus is difficult for people to recognize as an ear. Solution to Problem

The present invention has been conceived in view of the above-described problems and provides a skeletal structure for an artificial pinna that closely resembles the autologous pinna in appearance and has strength and flexibility.

More specifically, a skeletal structure for an artificial pinna according to the present invention includes:
a base portion formed in a flat shape with a fibrous structural body using a bioabsorbable material; and
a helix portion and an antihelix portion disposed on the front surface of the base portion.

Furthermore, a skeletal structure for an artificial cartilaginous tissue according to the present invention is formed by alternately stacking a first layer in which a fibrous structural body using a bioabsorbable material is formed in a wave shape and a second layer adhering closely to the first layer, the second layer being formed in a wave shape with a phase shift relative to the first layer.

Furthermore, a skeletal structure for an artificial cartilaginous tissue according to the present invention may be formed by alternately stacking a layer in which fibrous structural bodies using a bioabsorbable material are longitudinally arranged and a layer in which fibrous structural bodies are laterally arranged.

### Advantageous Effects of Invention

In the skeletal structure for the artificial pinna according to the present invention, the base portion, the helix portion, and the antihelix portion are formed with a fibrous structural body using the bioabsorbable material, making it possible to obtain these members as structural bodies with a large number of inter-fiber spaces. Thus, when these members are joined together, each member becomes very flexible, resists breakage even when experiencing large deformation, and can afterwards be restored to their original shapes.

Furthermore, the skeletal structure having a large number of inter-fiber spaces has the effect of facilitating the entry of cells or the like to the inside of the structure.

Furthermore, although the base portion is a flat member, the base portion is provided with a meander structure formed with the fibrous structural body using the bioabsorbable material. Thus, the base portion can deform in response to forces from multiple directions and afterwards restore itself.

Furthermore, the helix portion and the antihelix portion have a three-dimensional fiber structure formed with the fibrous structural bodies using the bioabsorbable material. Thus, even if the bioabsorbable material (so-called a plastic) has a high degree of hardness, the helix portion and the antihelix portion, as a whole, are flexible and can be restored to the original shape even if they are deformed.

Furthermore, the overall hardness of the three-dimensional fiber structure can be controlled by adjusting the distance between the fibrous structures that form a convoluted matrix in a single layer.

### Brief Description of Drawings

[Fig. 1] Fig. 1 is an assembly diagram of a skeletal structure for an artificial pinna according to the present invention.
[Fig. 2] Fig. 2 is a plan view of the skeletal structure.
[Fig. 3] Fig. 3 includes diagrams of the skeletal structure as viewed from different viewpoints.
[Fig. 4] Fig. 4 is an enlarged view of a two-dimensional elastic structure.
[Fig. 5] Fig. 5 includes diagrams illustrating a combined meander structure.
[Fig. 6] Fig. 6 includes diagrams illustrating another form of the two-dimensional elastic structure.
[Fig. 7] Fig. 7 includes diagrams describing a three-dimensional fiber structure (lattice type).
[Fig. 8] Fig. 8 is a perspective view of an end portion of the three-dimensional fiber structure (lattice type).
[Fig. 9] Fig. 9 includes diagrams illustrating another configuration of the three-dimensional fiber structure (braid type).
[Fig. 10] Fig. 10 includes diagrams illustrating the basic structure of the lattice type.
[Fig. 11] Fig. 11 includes diagrams illustrating the basic structure of the braid type.
[Fig. 12] Fig. 12 includes diagrams illustrating the skeletal structure including the C cartilage portion viewed from multiple viewpoints.
[Fig. 13] Fig. 13 includes diagrams describing a mechanical deformation test.

### Description of Embodiments

Hereinafter, a skeletal structure for an artificial pinna according to the present invention will be described with reference to the drawings. Note that the following description is intended to illustrate one embodiment and one example of the present invention, and the present invention is not limited to the following description. The following description can be modified without departing from the spirit of the present invention.

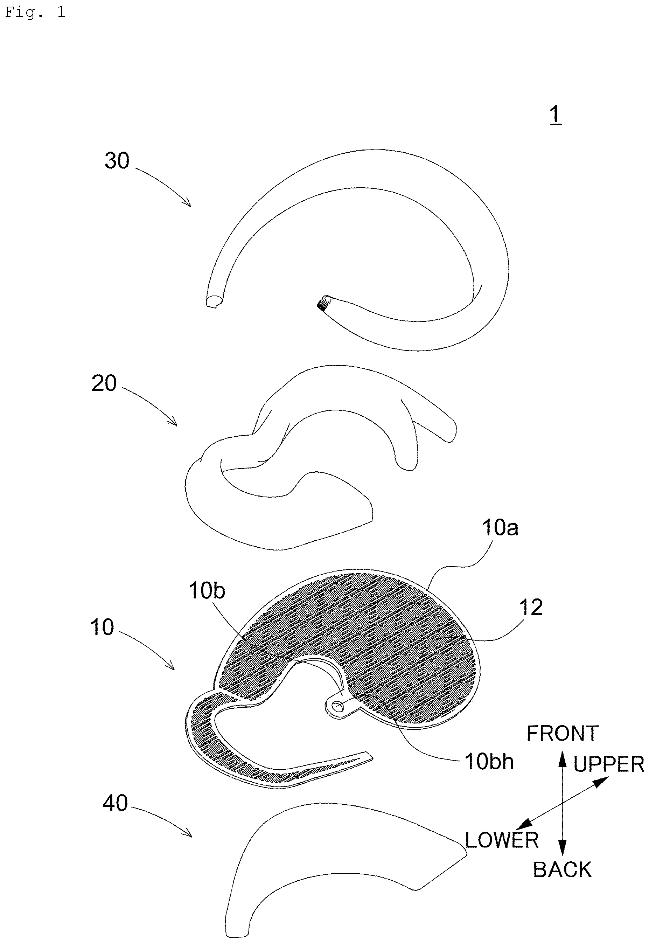

Fig. 1 illustrates an assembly diagram of a skeletal structure 1 of an(hereinafter simply referred to as "skeletal structure 1") according to the present invention. The skeletal structure 1 includes a helix portion 30, an antihelix portion 20, and a base portion 10. The skeletal structure 1 may further include a C cartilage portion 40. The helix portion 30 and the antihelix portion 20 are disposed on the front side of the planar base portion 10. Note that there is no particular difference in structure between the front and back of the base portion 10, and the terms "front" and "back" are used for the purpose of description. The C cartilage portion 40 is a member having an inclined surface as described below and is used for inclining the skeletal structure 1 with respect to a face on which the artificial pinna is to be formed.

Hereinafter, the side where the helix portion 30 is formed is referred to as "upper", and the opposite side is referred to as "lower". Furthermore, as described above, the side of the base portion 10 on which the helix portion 30 and the antihelix portion 20 are placed is referred to as "front", and the opposite side is referred to as the "back".

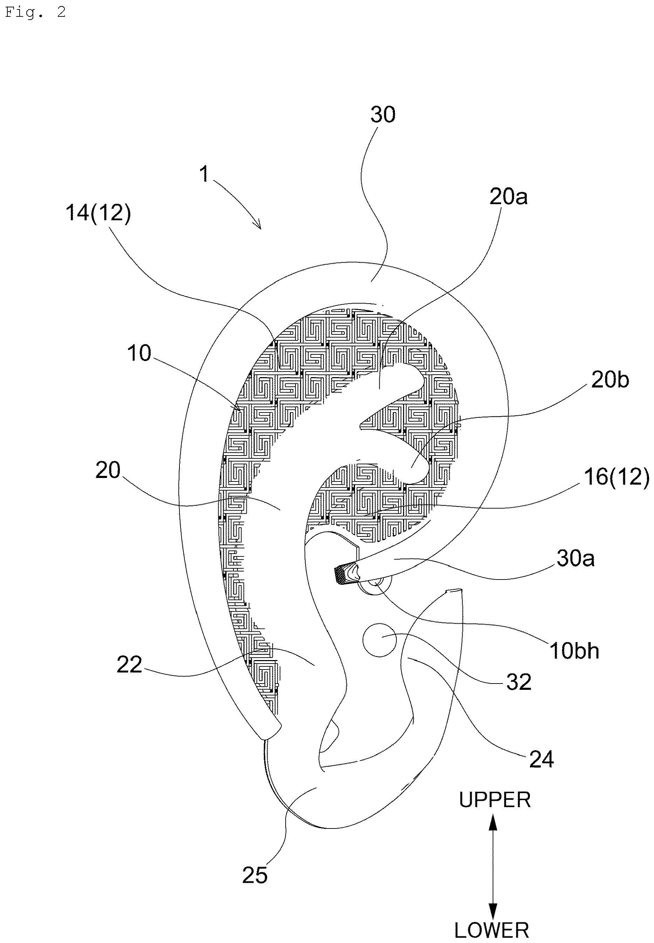

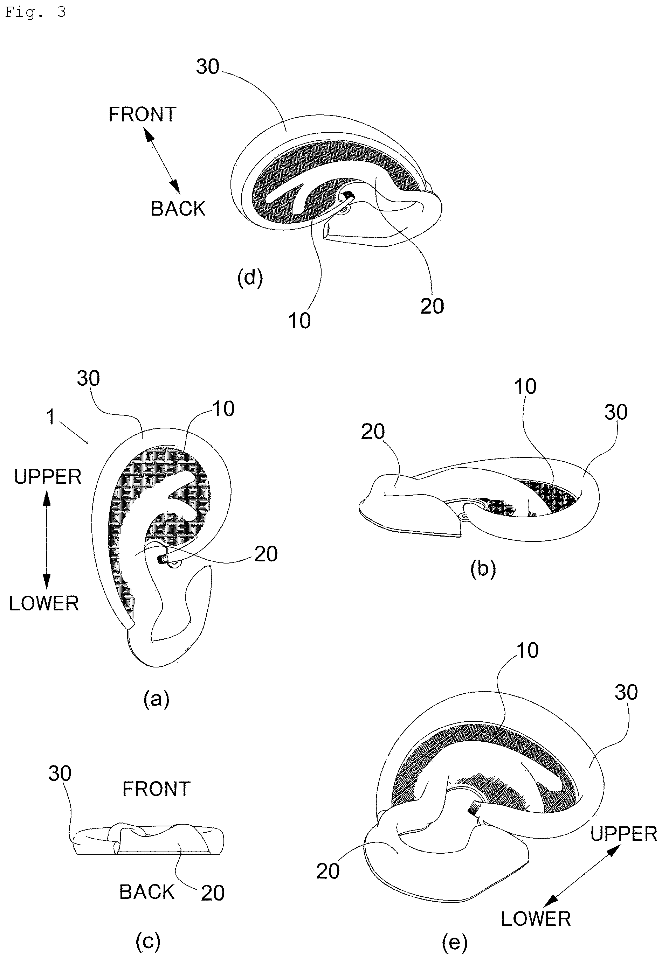

Fig. 2 illustrates a plan view in which the helix portion 30 and the antihelix portion 20 are placed on the front surface of the base portion 10. Furthermore, Fig. 3 illustrates diagrams of the skeletal structure 1 viewed from different viewpoints. Fig. 3(a) is a plan view, which is the same as Fig. 2. Fig. 3(b) is a perspective view of the skeletal structure 1 viewed from the right front side. Fig. 3(c) is a side view of the skeletal structure 1 viewed from the lower side. Fig. 3(d) is a perspective view of the skeletal structure 1 viewed from the upper back surface. Fig. 3(e) is a perspective view of the skeletal structure 1 viewed from the lower front surface.

First, referring to Figs. 1 to 3, the base portion 10 is an eggplant-shaped planar member. Fig. 3(c) shows that the back side is made flat. Referring to Fig. 1, a rim 10a is provided around the base portion 10. A bracket 10b having a for-fastening through hole 10bh may be provided on the rim 10a. A two-dimensional elastic structure 12 described below is formed inside the rim 10a.

Referring to Fig. 2, on the front surface of the base portion 10, the helix portion 30 and the antihelix portion 20 are connected. The helix portion 30 is formed along the rim 10a of the base portion 10, extending from a helical crus portion 30a at the upper part of a region 32 corresponding to the external auditory canal to an ear lobe portion 25. Note that the helical crus portion 30a may be extended to such a position where it covers the for-fastening through hole 10bh.

On the other hand, the antihelix portion 20 is disposed such that an antihelix upper leg portion 20a and an antihelix lower leg portion 20b at the upper end are disposed closely to the helical portion 30 near the center of the upper part of the base portion 10. Then, the antihelix portion 20 is extended to the lower side to form an antitragus portion 22 and further extended to form the ear lobe portion 25 and finally a tragus portion 24, in an integral manner. The helix portion 30 and the antihelix portion 20 are formed with a three-dimensional fiber structure 50 described below.

Due to the positional relationship of the base portion 10, the helix portion 30, and the antihelix portion 20, a scaphoid fossa portion 14 is covered by the base portion 10. Furthermore, a part of a concha portion 16 is covered by the base portion 10, and the concha portion 16 has a hollowed space that goes from the region 32 corresponding to the external auditory canal to the ear lobe portion 25.

### <Two-dimensional elastic structure>

In the present invention, the term "fibrous structural body using a bioabsorbable material" refers to a bioabsorbable material that is formed in a fiber-like shape. Examples thereof may include a structural body obtained by ejecting a gel-like or molten bioabsorbable material in an elongated shape through a nozzle, a structural body obtained by forming a granular bioabsorbable material into a fiber-like shape and then bundling such a material, and a structural body obtained by forming a plate-shaped bioabsorbable material into a shape that looks as if it is made of a fiber by removing unnecessary parts from the plate-shaped bioabsorbable material. Furthermore, the term "fiber-like shape" is not always used for a single fiber, and fibers formed with multiple fibers may also be called the "fibrous structural body using a bioabsorbable material". Note that the term "fibrous structural body using a bioabsorbable material" is also referred to as a "fibrous structure".

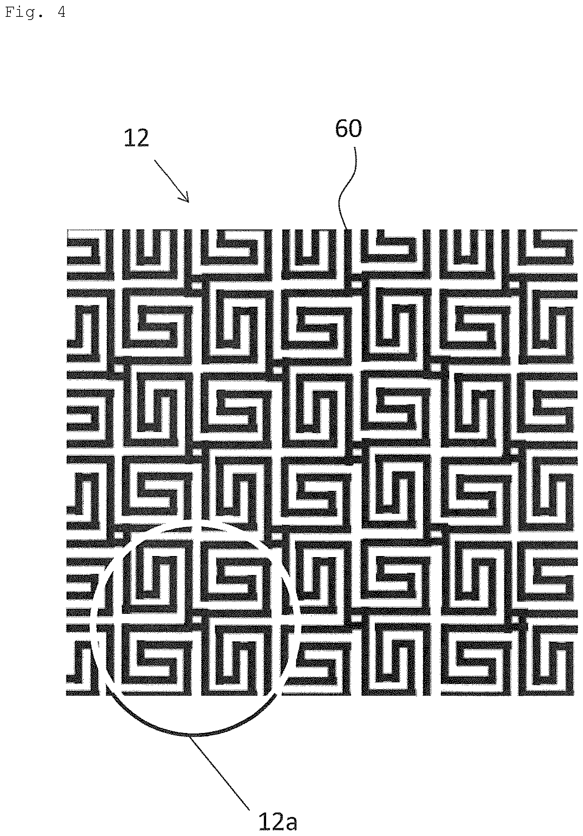

Fig. 4 illustrates an enlarged view of the two-dimensional elastic structure 12 that constitutes the inside of the rim 10a of the base portion 10. The two-dimensional elastic structure 12 is a planar member formed using a fibrous structure 60 of the bioabsorbable material, and achieves elasticity in the longitudinal direction and the lateral direction by repeatedly linking unit structures 12a. The constituting fibrous structure 60 preferably has a diameter of about 50 µm to 1 mm. Fig. 4 illustrates a combined meander structure as an example.

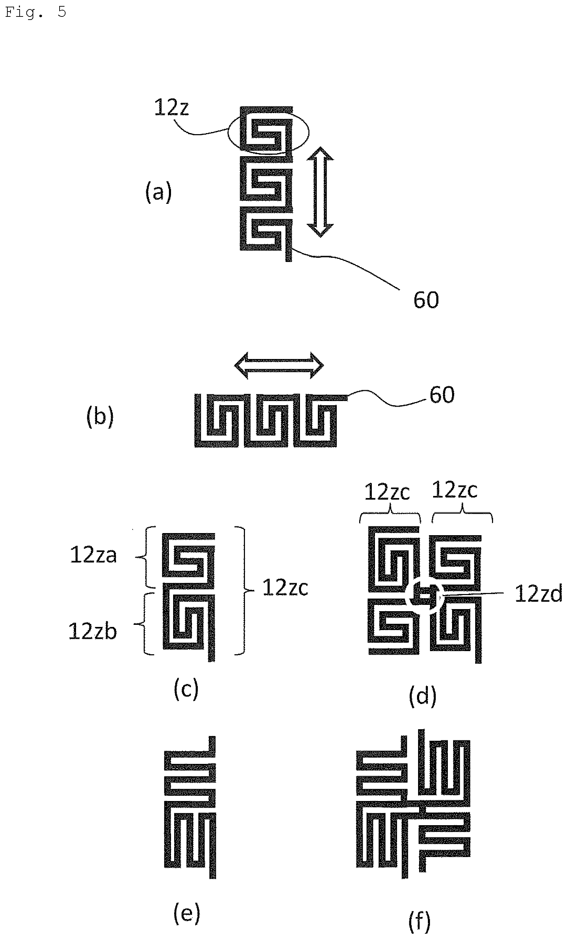

Referring to Fig. 5, the meander structure is a planar member that is formed by layering a pattern called a meander pattern as shown in Fig. 5(a), to give the structure a thickness of about 1 mm to 3 mm. The meander structure is a pattern originally called a Greek fret design, in which a single line is bent to create a spiral shape 12z, and this pattern is repeated continuously along a length direction of the line.

This pattern can be considered as a structure in which wire materials are assembled and shortened in the length direction. Thus, such a structure has elasticity both in the longitudinal direction and the lateral direction. In particular, the structure has a high stretching ability in the length direction. The meander structure arranged longitudinally (Fig. 5(a)) has a high stretching ability in the longitudinal direction indicated by an arrow, and the meander structure arranged laterally (Fig. 5(b)) has a high stretching ability in the lateral direction indicated by an arrow.

A combined meander structure (Fig. 5(d)) has a basic unit structure 12zc (Fig. 5(c)) that combines a layered structure of a unit pattern 12za in the longitudinal direction in Fig. 5(a) and a layered structure of a unit pattern 12zb in the lateral direction in Fig. 5(b). These basic unit structures are arranged in parallel and connected using a joint portion 12zd to obtain the combined meander structure. This structure corresponds to the area indicated by reference sign 12a in Fig. 4.

Note that the two-dimensional elastic structure 12 may be any structure obtained by two-dimensionally assembling and shortening the fibrous structures 60 in the longitudinal direction and the lateral direction, and arranging such fibrous structures 60 in a continuous manner. For example, a planar structure (Fig. 5(f)) may be obtained by using a unit structure shown in Fig. 5(e) that combines a structure with a pattern of longitudinally repeating rectangles and a structure with a pattern of laterally repeating rectangles, and connecting such unit structures using a joint portion.

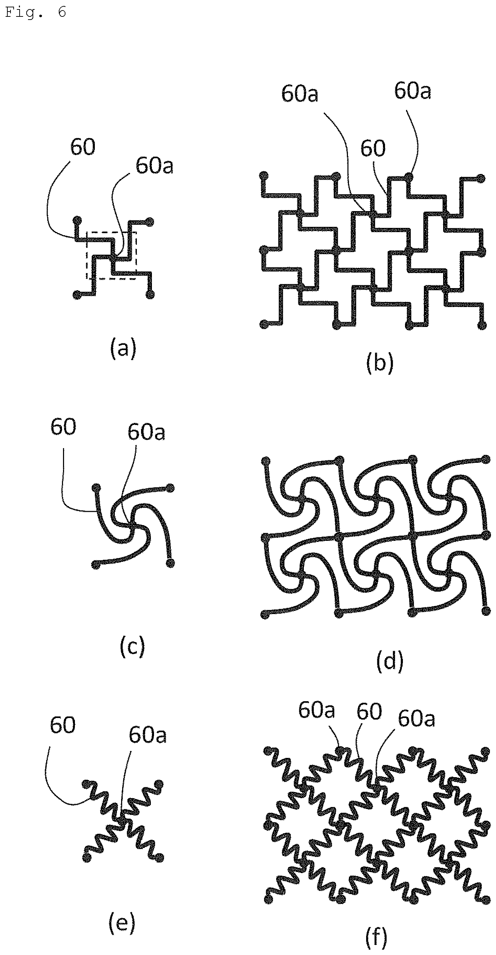

Fig. 6 illustrates another configuration of the two-dimensional elastic structure 12. Figs. 6(a) and 6(b) illustrate a case in which the fibrous structure 60 has a swastika pattern. In Fig. 6(a), the area surrounded by dotted lines has a swastika pattern. Fig. 6(b) is a repeated pattern of Fig. 6(a). Figs. 6(c) and 6(d) illustrate patterns that are slightly distorted from the swastika pattern. Furthermore, Figs. 6(e) and 6(f) illustrate patterns in which intersection points 60a of the fibrous structures 60 are connected by the wave-shaped fibrous structures 60.

In this manner, the two-dimensional elastic structure can be formed by making the length of the fibrous structure 60 between the intersection points 60a of the fibrous structure 60 longer than the shortest distance between the intersection points 60a.

### <Three-dimensional fiber structure>

A three-dimensional fiber structure 50 is a structure in which a solid shape is formed by stacking layers that are composed of twisted fibrous structures 60 of the bioabsorbable material. Herein, the fibrous structures 60 are twisted so as to pass through both a part of the outer periphery and the inside of the desired shape. This three-dimensional fiber structure 50 may be also called a "braid structure". Ensuring that adjacent layers in the stacking direction do not have the same pattern makes it possible to obtain a structure with a large number of spaces between the fibrous structures 60. Note that the thickness of the fibrous structure 60 is preferably about 50 µm to 1 mm in diameter.

Such a structure can provide high strength in the stacking direction and elasticity in the plane orthogonal to the stacking direction. Furthermore, another advantage is that, due to a large number of spaces between the fibrous structures 60, the cartilaginous tissue can easily penetrate inside the structure.

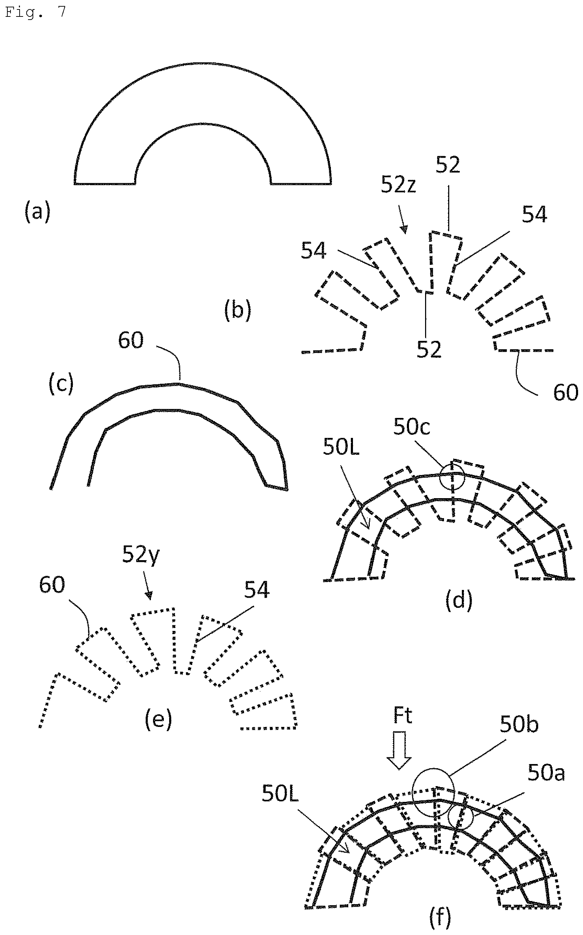

Fig. 7 illustrates an example of the three-dimensional fiber structure 50. The example is provided in which a solid structure has a bottom surface in a half-doughnut shape as shown in Fig. 7(a), and this shape is three-dimensionally (from the back to the front of the drawing) built into the solid structure using the three-dimensional fiber structure 50. In this case, as shown in Fig. 7(b), a layer is formed with the fibrous structure 60 passing through both a part 52 of an external shape and an inner portion 54. Note that Fig. 7(b) is a pattern of the first layer, Fig. 7(c) is a pattern of the second layer, and Fig. 7(e) is a pattern of the third layer.

It is preferable that each adjacent layer of this three-dimensional fiber structure 50 has a different pattern. This preference is due to the difficulty in creating spaces when the same patterns are used. In this example, the first layer (Fig. 7(b)) and the third layer (Fig. 7(e)) have the rectangular repeating structures with shift phases. That is, on the outer periphery, the first layer does not have the fibrous structure 60 in a portion 52z. However, the third layer has the fibrous structure 60 with a shifted phase (reference sign 52y) in the position corresponding to the portion 52z.
Note that the fibrous structures 60 passing through the inner portion 54 may overlap each other at the same position.

Furthermore, the three-dimensional fiber structure 50 may include a layer having a pattern present only in the outer periphery of the external shape or only in the inner portion 54. Fig. 7(c) shows a pattern that passes through only the inner portion 54 of the desired shape (Fig. 7(a)).

In a method for producing the three-dimensional fiber structure 50, a pattern shown in Fig. 7(b) is formed, on a base material, using the fibrous structure 60 of the bioabsorbable material. Next, a pattern shown in Fig. 7(c) is stacked as a second layer (Fig. 7(d)). Next, a pattern shown in Fig. 7(e) is formed on the layers in Fig. 7(d) (Fig. 7(f)).

When the three-dimensional fiber structure 50 is two-dimensionally viewed, a lattice 50L is formed by the longitudinal and lateral fibrous structures 60. Such a lattice 50L is preferably set to a size of about 10 µm × 10 µm to 1 mm × 1 mm square. This allows the cells and extracellular matrix to enter the structure.

Fig. 7(f) illustrates the resulting three layers formed in this manner. Reference signs 50a and 50b indicate areas where the fibrous structures 60 overlap each other in the first layer and the third layer.

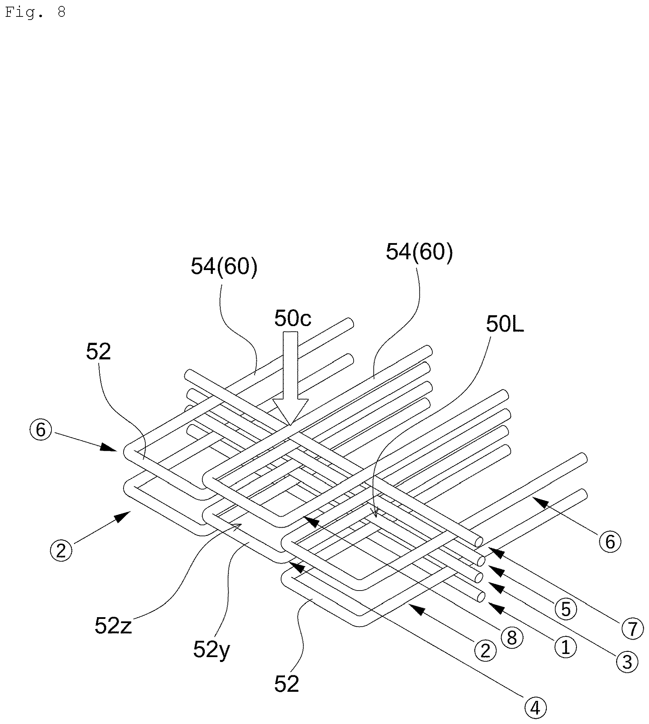

Fig. 8 illustrates a perspective view of a structure in which multiple layers of such patterns are stacked. This diagram shows how the patterns are stacked from the first layer to the eighth layer. Each layer is indicated by a number in a circle.

Viewing from the side, the sixth layer is sandwiched between the fourth layer and the eighth layer, resulting in the formation of a portion 52z where there is no fibrous structure 60. Above and below the portion 52z where there is no fibrous structure 60, portions 52y of the fibrous structures 60 with the shifted phases are formed.

As described above, when viewed from the side, the portion 52z without the fibrous structure 60 and the portion 52y of the fibrous structure 60 with the shifted phases are alternately formed, thereby forming a large number of spaces.

In this configuration, reference sign 50c indicates a part where each layer is stacked in a tight or intersectional manner based on the design. Such a part is called a "pseudo-column portion 50c". The three-dimensional fiber structure 50 may have such a pseudo-column portion 50c in which the plurality of layers are tightly stacked. At the intersection part, a melted fibrous structure 60 is placed as an upper layer is formed in the stacking direction, and thus the upper and lower layers are bonded together after the melted fibrous structure 60 cools down. When the fibrous structure 60 cools and hardens, the pseudo-column portion 50c gains compressive stress in an arrow direction (thickness direction) in accordance with material characteristics of the fibrous structure 60. That is, the pseudo-column portion 50c is firm and hardly deformed.

On the other hand, as shown in Fig. 7(f), the structure as a whole can have elasticity in response to a force Ft from the side direction. That is, the structure is soft, easily deformed, and easily restored.

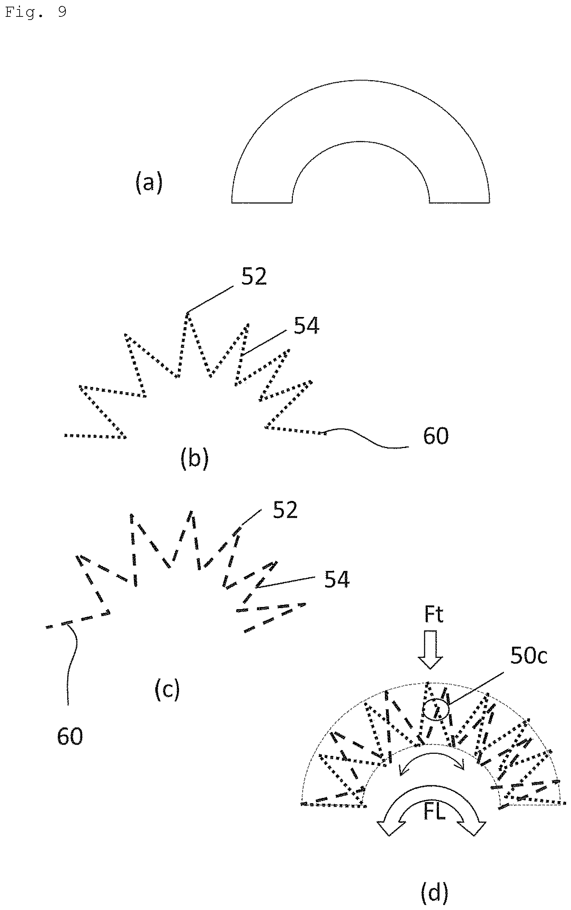

Fig. 9 illustrates another form of the three-dimensional fiber structure 50. Fig. 9 illustrates a case in which the inner portion 54 of the desired shape is formed in a triangular wave pattern, and layers having such a pattern are stacked. Note that although the triangular wave is illustrated in this example, any waveform shape other than the triangular wave may be used. Fig. 9(b) shows a first layer, and Fig. 9(c) shows a second layer. Each of these layers passes through the part 52 of the outer periphery and the inner portion 54 of the desired shape (Fig. 9(a)).

The part 52 of the external shape may be a point as shown in this case. When the first layer and the second layer with such a configuration are stacked, the pseudo-column portion 50c is formed in the inner portion 54 at a part where the first layer and the second layer intersect each other (Fig. 9(d)). Thus, even using this shape, compressive stress in the stacking direction is gained in accordance with the material characteristics of the fibrous structure 60, and has elasticity in an in-plane direction Ft and a direction FL (length direction), which is the direction in which the three-dimensional fiber structure 50 is formed.

In particular, when the triangular wave patterns are stacked, there is no part that is subjected to tension and compression in the side direction (indicated by an arrow). Thus, the elasticity is higher than that received by a pattern based on the rectangular wave as shown in Fig. 7. As a result, when the triangular wave pattern is used for the helix portion 30, the outer periphery of the pinna is easily deformed, making it possible to provide a texture closer to that of the actual pinna. Furthermore, this configuration allows expansion and contraction in the length direction FL because the intersection points of the first layer and the second layer are aligned along the direction FL, which is the direction in which the three-dimensional fiber structure 50 is formed.

Note that the in-plane elasticity of the three-dimensional fiber structure 50 can be lowered by adjusting the distance between the fibrous structures 60. That is, the three-dimensional fiber structure 50 is made to be difficult to deform.

### <Three-dimensional fiber structure (basic)>

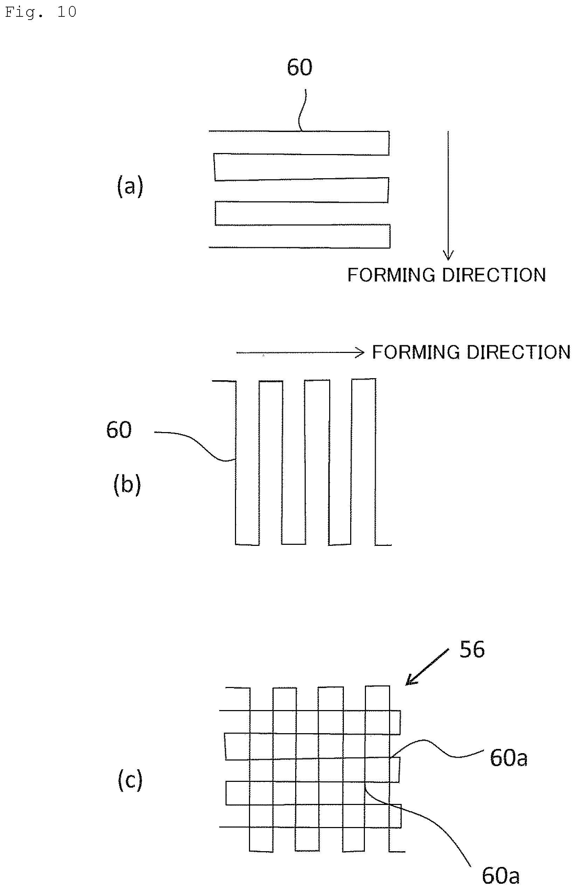

At a more basic level, the three-dimensional fiber structure 50 includes two types: an intersecting rectangular type (referred to as "lattice type 56") and a superimposed type of waveforms with shifted phases (referred to as "braid type 58"). Fig. 10 illustrates the basic structure of the lattice type 56. The lattice type 56 is constituted by overlapping a rectangular wave layer formed from top to bottom (Fig. 10(a)) and a rectangular wave layer formed from left to right (Fig. 10 (b)), both layers being formed with the fibrous structure 60 of the bioabsorbable material.

The lattice type 56 include intersection points 60a of the fibrous structures 60 in both the longitudinal direction and the lateral direction, making it difficult to deform the lattice type 56 in both the longitudinal and lateral directions. At the intersection point 60a, the upper and lower layers in the stacking direction are connected to each other (Fig. 10(c)). Since the intersection points 60a exist two-dimensionally, the rigidity becomes high in both the longitudinal direction and the lateral direction. Note that the structure can be extended in the height direction by repeating the above-mentioned two layers. The structure in Fig. 7 is an example of the lattice type 56.

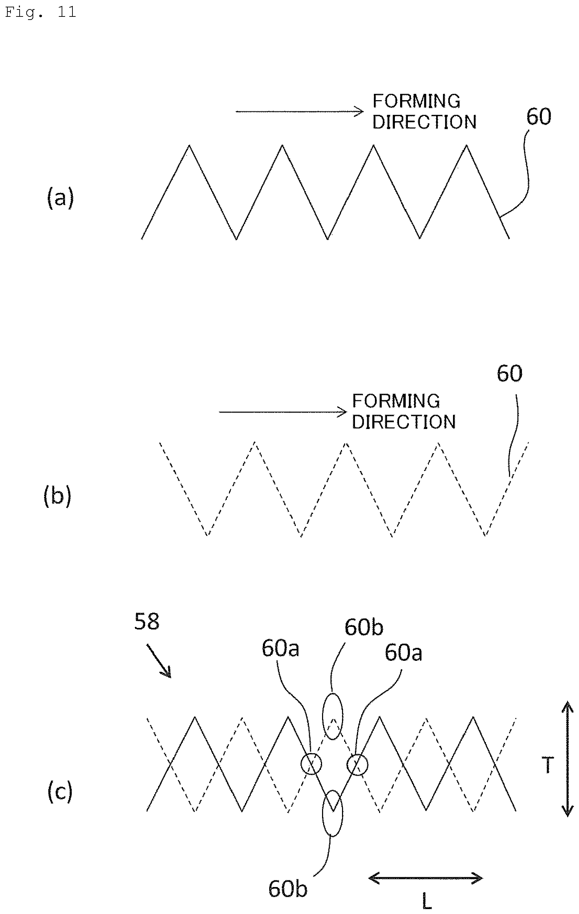

Fig. 11 illustrates a basic structure of the braid type 58. The braid type 58 (Fig. 11(c)) is formed by stacking, on a first layer (Fig. 11(a)) that is composed of wave-shaped fibrous structures 60 of the bioabsorbable material extending in one direction, a second layer (Fig. 11(b)) with phase-shifted wave shapes. The braid type 58 is excellent in elasticity in a length direction L and in a direction T orthogonal thereto since the intersection points 60a of the fibrous structures 60 between the upper and lower layers exist one-dimensionally. The term "one-dimensionally" described herein means that the intersection points 60a of the fibrous structures 60 in the layers adjacent in the stacking direction are aligned along the direction in which the three-dimensional fiber structure 50 is formed.

Furthermore, it can be said that the basic structure of the braid type 58 includes, between a pair of adjacent intersection points 60a, a loop portion 60b that does not contain an intersection point 60a. With this basic shape, even if the intersection points 60a are separated apart, the loop portion 60b is deformed to allow each part to follow the deformation of the entire structure. Fig. 9 is an example of the braid type 58.

It can be said that the lattice type 56 and the braid type 58 are very useful shapes as basic skeletons for cartilage formation. Note that the shape of each layer forming the lattice type 56 and the braid type 58 can be changed on the basis of strength design. Furthermore, when the number of stacked layers increases, the intersection points (connection points) 60a may be shifted slightly because the overall elasticity and flexibility can be adjusted by such a shift. Furthermore, as shown in Fig. 1, the skeletal structure for the artificial cartilage (also referred to as "artificial cartilaginous tissue") can be achieved by a combination of the skeletal structure 1 of the lattice type 56 and the skeletal structure 1 of the braid type 58.

As described above, elasticity is exhibited in the entirety of the skeletal structure 1 in which the helix portion 30 and the antihelix portion 20 having the three-dimensional fiber structure 50 are bonded on the base portion 10 having the two-dimensional elastic structure 12. Moreover, even if the skeletal structure 1 undergoes a large deformation, it can be restored to the original shape. That is, it is possible to obtain a skeletal structure 1 of the artificial pinna that has flexibility despite using the bioabsorbable material, which is basically a plastic resin material. The term "flexibility" described herein refers to the ability to restore the original shape without breaking even after a large deformation is applied.

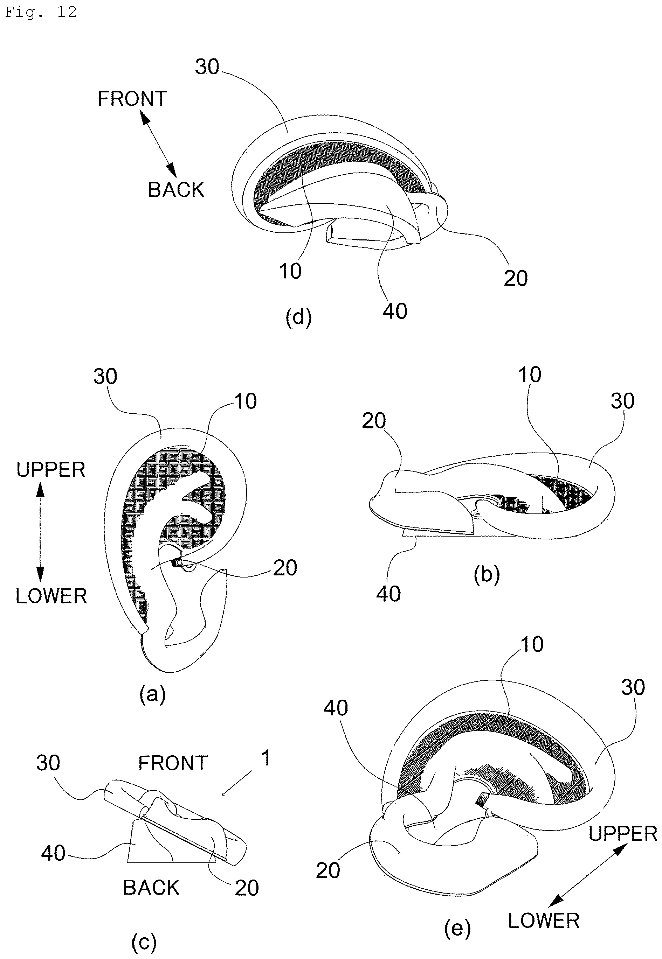

Fig. 12 illustrates a state in which the C cartilage portion 40 is bonded to the back surface of the base portion 10. The C cartilage portion 40 is used when the base portion 10 is angled with respect to the plane. Fig. 12(a) is a plan view, which is the same as Fig. 2. Fig. 12(b) is a perspective view of the skeletal structure 1 viewed from the right front side. Fig. 12(c) is a side view of the skeletal structure 1 viewed from the lower side. It can be understood that the C cartilage portion 40 gives an inclination to the skeletal structure 1 that connects the base portion 10, the helix portion 30, and the antihelix portion 20. Fig. 12(d) is a perspective view of the skeletal structure 1 viewed from the upper back surface. Fig. 12(e) is a perspective view of the skeletal structure 1 viewed from the lower front surface.

Note that the C cartilage portion 40 may be included in the skeletal structure 1. The C cartilage portion 40 may be formed with the three-dimensional fiber structure 50. However, since the C cartilage portion 40 is disposed between the skull and the pinna, its elasticity in the lateral direction does not have to be as high as that of the base portion 10, the helix portion 30, and the antihelix portion 20.

### <Material>

The base portion 10, the helix portion 30, the antihelix portion 20, and the C cartilage portion 40 can be all formed using the fibrous structure 60 made from a bioabsorbable material such as polyglycolide, polylactide (D, L, DL form), polycaprolactone, a glycolic acid-lactide (D, L, DL form) copolymer, a glycolic acid-ε-caprolactone copolymer, a lactide (D, L, DL form)-e-caprolactone copolymer, or poly(p-dioxanone). The fibrous structure 60 preferably has a diameter of about 50 µm to 1 mm.

A 3D printer can be suitably used for forming these components. In particular, the FDM (fused deposition modeling) method can be suitably used because the above-mentioned materials are thermoplastic. The SLS (selective laser sintering) method, the SLA (stereolithography) method, the DLP (digital light processing) method, and other methods are not excluded.

### <Artificial pinna>

The skeletal structure 1 is wrapped with bioabsorbable nonwoven fabric, and chondrocytes are seeded for culturing. After culturing, implantation surgery is performed on the temporal region of a patient. These steps can utilize known methods. For example, the following materials and methods described in PTL 3 can be suitably used.

Examples of a raw material for the bioabsorbable nonwoven fabric include polyglycolide, polylactide (D, L, DL form), polycaprolactone, a glycolic acid-lactide (D, L, DL form) copolymer, a glycolic acid-ε-caprolactone copolymer, a lactide (D, L, DL form)-ε-caprolactone copolymer, and poly(p-dioxanone). These materials may be used alone or in combination of two or more. Of these, polyglycolide or a lactide (D, L, DL form)-ε-caprolactone copolymer can be preferably used.

Furthermore, as the bioabsorbable nonwoven fabric, the following nonwoven fabric is preferably used: the above-mentioned material is formed into a thread with an average fiber diameter of 0.90 to 20.00 µm, and the resulting thread is formed into nonwoven fabric.

The skeletal structure 1 is wrapped with the nonwoven fabric made in this manner. The bioabsorbable nonwoven fabric is only required to cover at least the spaces in the skeletal structure 1. However, it is preferable that the bioabsorbable nonwoven fabric covers the entire structure. Furthermore, it is preferable that the bioabsorbable nonwoven fabric wraps the skeletal structure 1 as tightly as possible. The skeletal structure 1 according to the present invention, in which the scaphoid fossa portion 14 is filled with the base portion 10, can suitably support the bioabsorbable nonwoven fabric.

Auricular chondrocytes can be obtained as follows. The pinna is obtained from a human or an animal. After removing the skin, connective tissues, and perichondrium, the pinna is cut into small pieces of approximately 5 mm × 5 mm and then treated with collagenase to isolate auricular chondrocytes. The isolated auricular chondrocytes may be used as is or may be used after being propagated in culture.

A method of seeding the auricular chondrocytes onto the bioabsorbable nonwoven fabric is not particularly limited, and a conventionally known seeding method can be used. The seeding density during seeding is not particularly limited. However, a lower limit is preferably 2.0 × 10⁷ cells/cm² and an upper limit is preferably 1.0 × 10⁸ cells/cm². If the cell seeding density is less than 2.0 × 10⁷ cells/cm², it may take time to form pinna cartilaginous tissues with sufficient thickness and mechanical strength. Even if the cells are seeded at more than 1.0 × 10⁸ cells/cm², no further effect is observed. The lower limit of the cell seeding density is more preferably 5.0 × 10⁷ cells/cm².

As a culture medium for culturing, for example, a serum-containing medium prepared by adding about 1 to 10% by weight of fetal bovine serum to a general culture medium such as MEM or DMEM can be used.

The artificial pinna obtained by culturing the chondrocytes on the bioabsorbable nonwoven fabric wrapped around the skeletal structure 1 in this manner is implanted inside the body, making it possible to regenerate the pinna cartilaginous tissues with sufficient strength and elasticity.

### Examples

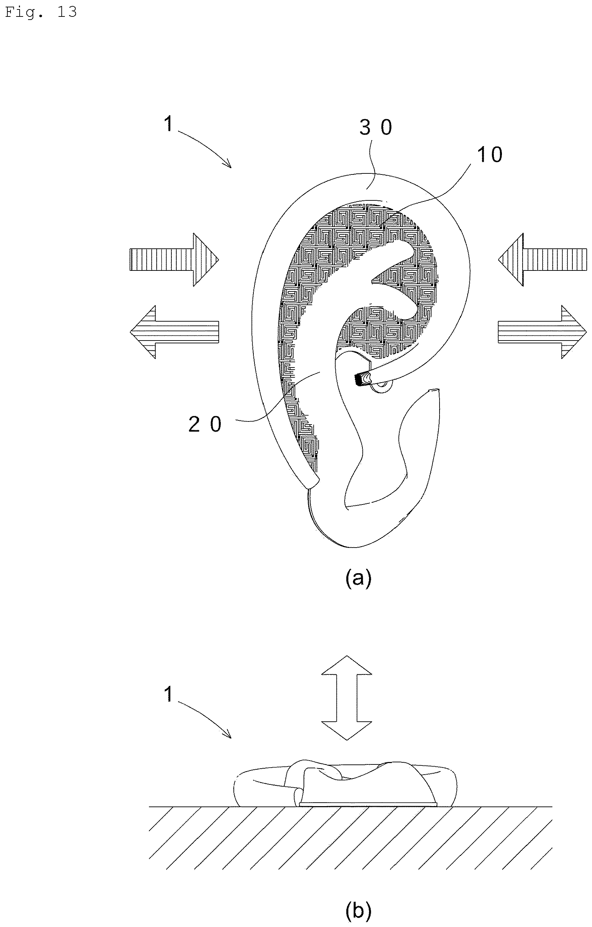

As shown in Fig. 13, the skeletal structure 1 in which the helical portion 30 and the antihelix portion 20 were bonded by thermal welding on the base portion 10 made from polycaprolactone was subjected to a mechanical deformation test in the thickness direction and in the plane direction. The base portion 10 was formed with a line width of 250 µm, a line spacing of 500 µm, and a thickness of 2 mm. The helix portion 30 was formed by stacking 15 layers of the fibrous structures 60 with a diameter of 300 µm formed in a triangular wave shape (based on Fig. 9) with a pitch of about 1 mm. Furthermore, the antihelix portion 20 was formed by stacking 25 layers formed in a rectangular wave shape (based on Fig. 7) with a pitch of 2 to 10 mm. These members were bonded by thermal welding to produce the skeletal structure 1 having the shape shown in Fig. 13(a).

In the mechanical deformation test in the lateral direction in Fig. 13(a), a stress/strain test that repeated 5 cycles of displacement with a displacement of 10 mm was performed using a commonly used stress/strain measuring instrument to determine a recovery rate based on a ratio of dimensions before and after the compression test. As a result, a force of approximately 20 N (approximately 2 kg weight) was required to displace the structure by 10 mm in the lateral direction, and the recovery rate was 99.6%.

This result showed that the structure did not break when subjected to a force of approximately 20 N and was able to be deformed by 10 mm in the lateral direction, and furthermore the structure showed the characteristic of almost completely restoring the original shape.

The mechanical deformation test in the thickness direction in Fig. 13(b) was performed in the same manner as in Fig. 13(a). A stress of 150 N was applied repeatedly for 5 cycles using the stress/strain measuring instrument to examine a recovery rate of the structure. As a result, a stress of 150 N caused a displacement of approximately 1 mm in the thickness direction, and the recovery rate was 98.8%.

This result showed that the structure did not break even when subjected to a force of approximately 15 kg weight and was able to be deformed by approximately 1 mm in the thickness direction, and furthermore the structure showed the characteristic of almost completely restoring the original shape when the stress was released.

Since the human head weighs about 5 kg, even if the weight of the head is directly applied to this skeletal structure 1 during sleeping in the lateral position, the skeletal structure 1 of the artificial pinna according to the present invention is restored to the original shape without breaking.

Furthermore, even if a force is applied from the lateral direction, the skeletal structure 1 can be displaced by as large as 10 mm, and even so, the skeletal structure 1 can be restored to the original shape.

### Industrial applicability

The skeletal structure 1 of the artificial pinna according to the present invention can be suitably used as a cell scaffolding material when the lost pinna is reconstructed by the implant-type regeneration therapy.

### Reference Signs List

1 skeletal structure
10 base portion
10a rim
10b bracket
10bh for-fastening through hole
12 two-dimensional elastic structure
12a unit structure
12za unit pattern
12zb unit pattern
12zc basic unit structure
12z spiral shape
14 scaphoid fossa portion
16 concha portion
20 antihelix portion
20a antihelix upper leg portion
20b antihelix lower leg portion
22 antitragus portion
24 tragus portion
25 ear lobe portion
30 helix portion
30a helical crus portion
32 region corresponding to external auditory canal
40 C cartilage portion
50 three-dimensional fiber structure
50c pseudo-column portion
52 part of external shape
54 inner portion
56 lattice type
58 braid type
60 fibrous structure
60a intersection point

## Claims

1. A skeletal structure for an artificial pinna comprising:
a base portion formed in a flat shape with a fibrous structural body using a bioabsorbable material; and
a helix portion and an antihelix portion disposed on a front surface of the base portion.

2. The skeletal structure for an artificial pinna according to claim 1, wherein the base portion is a planar member having a two-dimensional elastic structure formed with the fibrous structural body using the bioabsorbable material.

3. The skeletal structure for an artificial pinna according to claim 1 or 2, wherein the helix portion and the antihelix portion each have a three-dimensional fiber structure formed with a fibrous structural body using a bioabsorbable material.

4. The skeletal structure for an artificial pinna according to any one of claims 1 to 3, wherein the helix portion and the antihelix portion are welded to the base portion.

5. The skeletal structure for an artificial pinna according to any one of claims 1 to 4, further comprising a C cartilage portion disposed on a back surface of the base portion.

6. An artificial pinna comprising:
the skeletal structure for an artificial pinna according to any one of claims 1 to 5;
a bioabsorbable nonwoven fabric that covers the skeletal structure; and
chondrocytes that are cultured on the bioabsorbable nonwoven fabric.

7. A skeletal structure for an artificial cartilaginous tissue formed by alternately stacking a first layer in which a fibrous structural body using a bioabsorbable material is formed in a wave shape and a second layer adhering closely to the first layer, the second layer being formed in a wave shape with a phase shift relative to the first layer.

8. A skeletal structure for an artificial cartilaginous tissue formed by alternately stacking a layer in which fibrous structural bodies using a bioabsorbable material are longitudinally arranged and a layer in which fibrous structural bodies are laterally arranged.

9. A skeletal structure for an artificial cartilaginous tissue formed with the skeletal structure for an artificial cartilaginous tissue according to claim 7 and the skeletal structure for an artificial cartilaginous tissue according to claim 8.
